# EUROPEAN PATENT APPLICATION

(11) **EP 4 813 436 A1**
(43) Date of publication of application: **30.09.2026**
(21) Application number: 24933390.7
(22) Date of filing: 03.04.2024
(51) Int. Cl.: A61M 25/10

(54) **THREE-CAVITY MICROCATHETER**

(71) Applicant: Insight Lifetech Co., Ltd., Shenzhen, Guangdong 518000 (CN)
(72) Inventor: SHI, Zhengtao, Shenzhen, Guangdong 518000 (CN); ZHANG, Pengtao, Shenzhen, Guangdong 518000 (CN)
(74) Representative: Huang, Liwei
(86) International application number: PCT/CN2024/085923
(87) International publication number: WO 2025/208435

(57) **Abstract**

The present disclosure relates to the technical field of medical devices, and particularly relates to a three-lumen microcatheter, comprising: a first tube body, wherein a guide wire lumen is formed inside the first tube body; a second tube body; a third tube body; the first tube body has at least one guide wire outlet formed in a side wall of a distal end of the first tube body, and the guide wire outlet communicating with the guide wire lumen; wherein the second tube body, a distal portion of the first tube body and a distal portion of the third tube body are arranged side by side in a flattened configuration, when the three-lumen microcatheter enters a subintimal space, at least one guide wire outlet faces a vascular intima and/or a media; and a positioning marker, wherein the second tube body and/or the third tube body is provided with the positioning marker, and the positioning marker is used to position the guide wire outlet of the first tube body. The solution provided by the present disclosure can realize directional puncture for a puncture guide wire to return from a subintimal space to a true vascular lumen, and improves a success rate of a single operation.

## Description

### TECHNICAL FIELD

The present disclosure relates to the technical field of medical devices and particularly relates to a three-lumen microcatheter.

### BACKGROUND

Chronic Total Occlusion (CTO) lesions represent the final barrier of Percutaneous Coronary Intervention (PCI) and remain the most challenging procedure for interventional cardiologists. A standardized procedural framework for recanalizing Chronic Total Occlusion (CTO) lesions has been established across Europe, the Americas and the Asia-Pacific region alike. In China, four primary recanalization strategies are adopted for CTO-PCI: Antegrade Wire Escalation (AWE), Retrograde Wire Escalation (RWE), Antegrade Dissection and Re-entry (ADR), and Retrograde Dissection and Re-entry (RDR).
Approximately 70% of CTO lesions can be successfully recanalized using antegrade wire escalation (including parallel wire technique). When combined with retrograde wire escalation, the procedural success rate for CTO lesions rises to 85%-90%. If recanalization cannot be achieved via antegrade or retrograde wire escalation pathways, the Antegrade Dissection and Re-entry (ADR) technique serves as a salvage strategy to further improve the success rate of CTO recanalization.

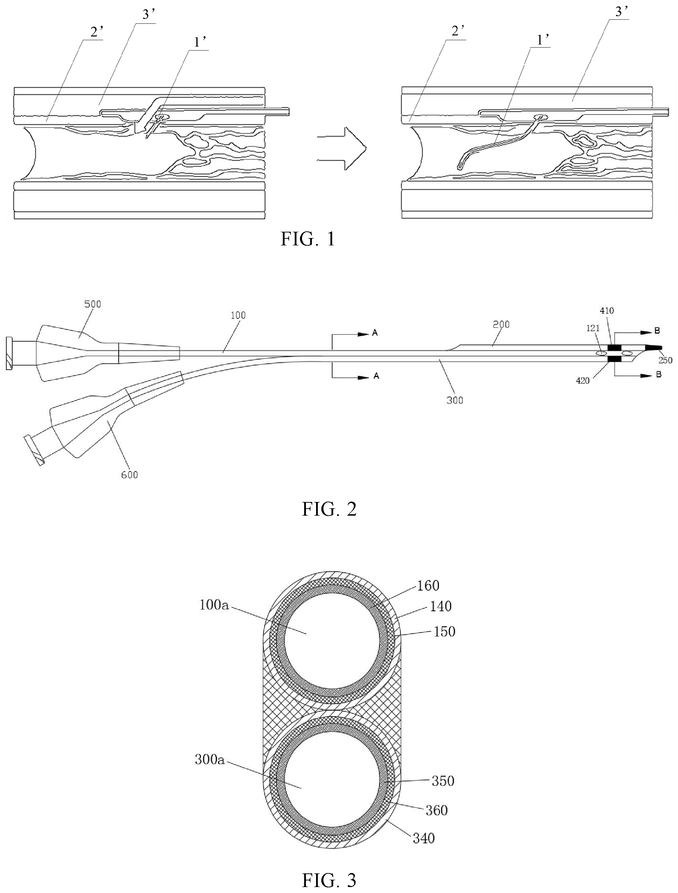

Antegrade Dissection and Re-entry (ADR) refers to a procedural strategy where devices advance along an antegrade guide wire within the true lumen, traverse the subintimal space of the occluded segment to reach a distal landing zone of the lesion, and a guide wire is then used under device assistance to puncture back into the true vascular lumen. During the procedure, as shown in Figure 1, a catheter (such as CrossBoss) bluntly dissects the subintimal tissue at the lesion site. A puncture guide wire 1' crosses the CTO segment via a false lumen outside the vessel and re-enters the true lumen with assistance from other devices. Figure 1 illustrates the process in which the puncture guide wire 1' positioned between a vascular intima 2' and a media 3' punctures back into the true vessel lumen.

Catheters commonly used in current ADR procedures exhibit poor trackability, which may prevent the puncture guide wire 1' from re-entering the true lumen and result in procedural failure. In addition, the catheter needs to perform multiple manipulations within the intima including balloon pre-dilation, expansion, aspiration and puncture, leading to complex procedural steps that hinder widespread clinical adoption.

### SUMMARY

In view of the above, the present disclosure provides a three-lumen microcatheter to solve the problem that a puncture guide wire is difficult to re-enter a true vascular lumen.

The present disclosure provides a three-lumen microcatheter, comprising: a first tube body, wherein a guide wire lumen is formed inside the first tube body; a second tube body; a third tube body; the first tube body has at least one guide wire outlet formed in a side wall of a distal end of the first tube body, and the guide wire outlet communicating with the guide wire lumen; wherein the second tube body, a distal portion of the first tube body and a distal portion of the third tube body are arranged side by side in a flattened configuration, when the three-lumen microcatheter enters a subintimal space, at least one guide wire outlet faces a vascular intima and/or a media; and a positioning marker, wherein the second tube body and/or the third tube body is provided with the positioning marker.

In one embodiment, axes of the first tube body, the second tube body and the third tube body are parallel to each other, and the first tube body, the second tube body and the third tube body lie in a same plane.

In one embodiment, there are two the guide wire outlets, the two guide wire outlets are formed on opposite side walls of the first tube body, and axes of the two guide wire outlets are perpendicular to the plane.

In one embodiment, the two guide wire outlets are spaced apart along an axial direction of the first tube body; and the positioning marker is arranged opposite to an area between the two guide wire outlets.

In one embodiment, the positioning marker comprises:
a first radiopaque marker arranged on the second tube body; and
a second radiopaque marker arranged on the third tube body, wherein the first radiopaque marker and the second radiopaque marker correspond to the two guide wire outlets, respectively, and the first radiopaque marker cooperates with the second radiopaque marker to position the two guide wire outlets.

In one embodiment, the first radiopaque marker and the second radiopaque marker are symmetrically arranged relative to the first tube body.

In one embodiment, the positioning marker is a radiopaque marker ring or a radiopaque marker wire.

In one embodiment, the first tube body comprises a proximal tube segment and a distal tube segment connected to each other, and the guide wire outlet is arranged in a side wall of the distal tube segment; and

the third tube body comprises a proximal tube body and a distal tube body connected to each other, the proximal tube body is arranged side by side with the proximal tube segment, and the distal tube body, the distal tube segment and the second tube body are arranged side by side in a flattened configuration.

In one embodiment, distal ends of the first tube body, the second tube body and the third tube body are arranged in a stepped arrangement.

In one embodiment, the second tube body and the third tube body are disposed on two sides of the first tube body, and a distal end of the second tube body is located at an distal-most position of the stepped arrangement, or a distal end of the third tube body is located at the distal-most position of the stepped arrangement.

In one embodiment, when the distal end of the second tube body is located at the distal-most position of the stepped arrangement, a cross-sectional area of the distal end of the second tube body gradually decreases in a direction from a proximal end to a distal end, and the distal ends of the first tube body and the third tube body each form an inclined surface; or
when the distal end of the third tube body is located at the distal-most position of the stepped arrangement, a cross-sectional area of the distal end of the third tube body gradually decreases in a direction from a proximal end to a distal end, and the distal ends of the first tube body and the second tube body each form an inclined surface.

In one embodiment, the second tube body and the third tube body are respectively disposed on two sides of the first tube body, and a distal end of the first tube body protrudes beyond distal ends of the second tube body and the third tube body.
In one embodiment, the first tube body further comprises a stress relief tube segment, one end of the stress relief tube segment is connected to the proximal tube segment, and another end is configured to connect to a catheter hub, and inner spaces of the stress relief tube segment, the proximal tube segment and the distal tube segment communicate with each other to form the guide wire lumen; and/or
the third tube body further comprises a stress relief tube body, one end of the stress relief tube body is connected to the proximal tube body, and another end is configured to connect to a catheter hub, and inner spaces of the stress relief tube body, the proximal tube body and the distal tube body communicate with each other to form a lumen.

Compared with balloon catheters in related art, the three-lumen microcatheter of the present disclosure enables directional puncture for a puncture guide wire to return from a subintimal space to a true vascular lumen. For example, when the three-lumen microcatheter enters an intramembranous space (the space formed after separation of an intima and a media), the first tube body, the second tube body and the third tube body are arranged side by side in a flattened configuration. Under compressive force from the intima and the media, the three-lumen microcatheter deflects such that at least one guide wire outlet faces the vascular intima and/or the media. When the guide wire outlet faces the intima, a puncture guide wire may extend out of the guide wire outlet through the first guide wire lumen, penetrate the intima, and re-enter the true vascular lumen. Even if the three-lumen microcatheter is delivered to an unfavorable position and orientation, that is, the guide wire outlet fails to face the vascular intima with a certain deviation, the configuration of the three-lumen microcatheter and the reinforcing layer (spring and/or braided layer) provided on the tube bodies allow proper torsion to orient the guide wire outlet toward the intima, which raises the success rate for the puncture guide wire to re-enter the true vascular lumen and thus maximizes the success rate of a single operation. It can be seen that, unlike balloon catheters in related art which require complicated operations including balloon pre-dilation, expansion and aspiration, the three-lumen microcatheter of the present disclosure is easy to operate with simplified procedural steps, facilitating wide promotion and clinical application. In addition, in the present disclosure, the parallel arrangement of the first tube body, the second tube body and the third tube body of the three-lumen microcatheter enables rapid advancement into the subintimal area of a lesion and rapid withdrawal, delivering favorable trackability.

It should be understood that the above-mentioned general description and the subsequent detailed description are only exemplary and explanatory, and cannot limit the disclosure.

### BRIEF DESCRIPTION OF DRAWINGS

By describing the exemplary embodiments of the present disclosure in more detail with reference to the drawings, the above and other objectives, features and advantages of the present disclosure will become more apparent. In the exemplary embodiments of the present disclosure, the same reference numerals usually represent the same components.
FIG. 1 is a schematic diagram illustrating an Antegrade Dissection and Re-entry procedure in the related art.
FIG. 2 is a structural schematic diagram of a three-lumen microcatheter according to one embodiment of the present disclosure.
FIG. 3 is a cross-sectional view taken along line A-A of FIG. 2.
FIG. 4 is a cross-sectional view taken along line B-B of FIG. 2.
FIG. 5 is a structural schematic diagram of a distal portion of the three-lumen microcatheter shown in FIG. 2.
FIG. 6 is a structural schematic diagram of a three-lumen microcatheter according to another embodiment of the present disclosure.
FIG. 7 is a cross-sectional view taken along line C-C of FIG. 6.
FIG. 8 is an overall cross-sectional view of a three-lumen microcatheter according to another embodiment of the present disclosure.
FIG. 9 is a three-dimensional structural schematic diagram of a distal portion of the three-lumen microcatheter shown in FIG. 8.
FIG. 10 is a cross-sectional view of a distal portion of the three-lumen microcatheter shown in FIG. 8.
FIG. 11 is an overall cross-sectional view of a three-lumen microcatheter according to another embodiment of the present disclosure.
FIG. 12 is a three-dimensional structural schematic diagram of a distal portion of the three-lumen microcatheter shown in FIG. 11.
FIG. 13 is a cross-sectional view of a distal portion of the three-lumen microcatheter shown in FIG. 11.
FIG. 14 is a schematic diagram showing a first state where the three-lumen microcatheter provided in an embodiment of the present disclosure enters an intramembranous vascular space.
FIG. 15 is a schematic diagram showing a second state where the three-lumen microcatheter provided in an embodiment of the present disclosure enters an intramembranous vascular space.
FIG. 16 is a cross-sectional view of part of a first tube body of the three-lumen microcatheter provided in an embodiment of the present disclosure.
FIG. 17 is an overall cross-sectional view of a three-lumen microcatheter according to another embodiment of the present disclosure.

### DETAILED DESCRIPTION

Embodiments of the present disclosure will be described in greater detail below with reference to the drawings. Although embodiments of the present disclosure are shown in the accompanying drawings, it should be understood that the present disclosure may be implemented in various forms and shall not be limited to the embodiments set forth herein. Rather, these embodiments are provided to render the present disclosure thorough and complete, and to fully convey the scope of the present disclosure to those skilled in the art.

Terms used in the present disclosure are merely for describing specific embodiments and are not intended to limit the present disclosure. Singular forms such as "a", "the" and "said" used in the present disclosure and the appended claims are also intended to cover plural forms unless the context clearly indicates otherwise. It shall further be understood that the term "and/or" as used herein refers to and encompasses any and all possible combinations of one or more of the associated listed items.

It should be appreciated that although terms "first", "second", "third" and the like may be adopted in the present disclosure to describe various pieces of information, such information shall not be restricted by these terms. These terms are only used to distinguish information of the same type from one another. For example, without departing from the scope of the present disclosure, first information may also be referred to as second information, and similarly, second information may also be referred to as first information. Accordingly, features defined by "first" and "second" may explicitly or implicitly include one or more such features. In the description of the present invention, the meaning of "a plurality of" is two or more, unless otherwise clearly and specifically defined.

The technical solutions of the embodiments of the present disclosure will be described in detail below with reference to the drawings.

Referring to FIG. 2 to FIG. 5, a three-lumen microcatheter according to one embodiment of the present disclosure includes a positioning marker, a first tube body 100, a second tube body 200 and a third tube body 300. A guide wire lumen 100a is formed inside the first tube body 100, and the guide wire lumen 100a is configured to accommodate a puncture guide wire. At least one guide wire outlet 121 is opened in a side wall at a distal end of the first tube body 100, the at least one guide wire outlet 121 communicates with the guide wire lumen 100a, and the puncture guide wire can extend out of the first tube body 100 through the guide wire outlet 121. It should be noted that the distal end mentioned in the embodiments of the present disclosure refers to the right end shown in FIG. 2.

The second tube body 200, a distal portion of the first tube body 100 and a distal portion of the third tube body 300 are arranged side by side in a flattened configuration, when the three-lumen microcatheter enters a subintimal space, at least one guide wire outlet 121 faces a vascular intima A and/or a media B, the subintimal space is a space between a vascular intima and a vascular media.

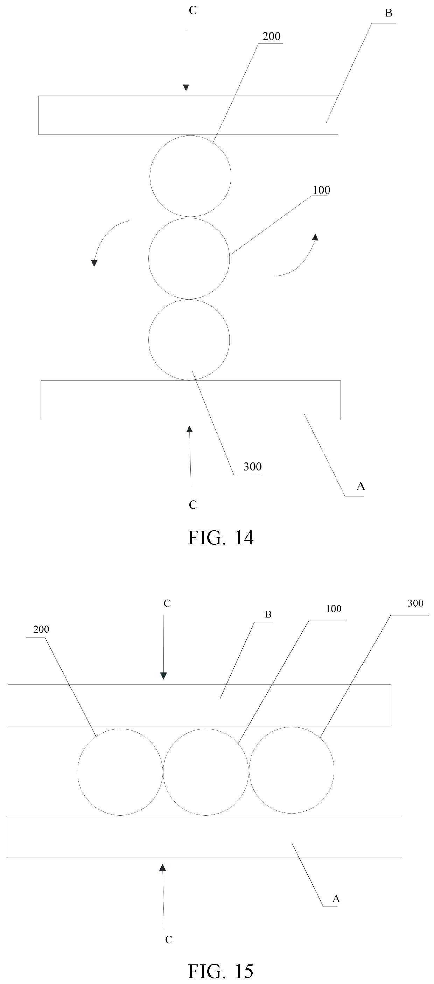

It should be noted that, with reference to FIG. 14 and FIG. 15, the intramembranous vascular space mentioned in this embodiment refers to a space formed after separation of the vascular intima A and the media B.

The second tube body 200 and/or the third tube body 300 are provided with positioning markers, which are used to position the guide wire outlet 121 of the first tube body 100 and facilitate extending the puncture guide wire out of the guide wire outlet 121 during surgery. In the illustrated embodiment, the positioning markers include a first radiopaque marker 410 and a second radiopaque marker 420 disposed on the second tube body 200 and the third tube body 300 respectively. In other embodiments, the positioning marker may be disposed only on the second tube body 200 or the third tube body 300.

Compared with balloon catheters in related art, the three-lumen microcatheter of the present disclosure enables directional puncture for a puncture guide wire to return from a subintimal space to a true vascular lumen. For example, when the three-lumen microcatheter enters an intramembranous space (the space formed after separation of an intima A and a media B), the first tube body 100, the second tube body 200 and the third tube body 300 are arranged side by side in a flattened configuration. Under compressive force from the intima and the media, the three-lumen microcatheter deflects such that at least one guide wire outlet 121 faces the vascular intima A and/or the media B. When the guide wire outlet 121 faces the intima A, a puncture guide wire may extend out of the guide wire outlet through the first guide wire lumen 100a, penetrate the intima, and re-enter the true vascular lumen. Even if the three-lumen microcatheter is delivered to an unfavorable position and orientation, that is, the guide wire outlet 121 fails to face the vascular intima with a certain deviation, the configuration of the three-lumen microcatheter and the reinforcing layer (spring and/or braided layer) provided on the tube bodies allow proper torsion to orient the guide wire outlet 121 toward the intima, which raises the success rate for the puncture guide wire to re-enter the true vascular lumen and thus maximizes the success rate of a single operation. It can be seen that, unlike balloon catheters in related art which require complicated operations including balloon pre-dilation, expansion and aspiration, the three-lumen microcatheter of the present disclosure is easy to operate with simplified procedural steps, facilitating wide promotion and clinical application. In addition, in the present disclosure, the parallel arrangement of the first tube body, the second tube body and the third tube body of the three-lumen microcatheter enables rapid advancement into the subintimal area of a lesion and rapid withdrawal, delivering favorable trackability.

In some embodiments, axes of the first tube body 100, the second tube body 200 and the third tube body 300 are parallel to each other, and the first tube body 100, the second tube body 200 and the third tube body 300 lie in a same plane, so that the three-lumen microcatheter deflects more easily inside an intramembranous vascular space.

There are two guide wire outlets 121, and the two guide wire outlets 121 are formed on opposite side walls of the guide wire lumen 100a. The axes of the two guide wire outlets 121 are perpendicular to the plane where the first tube body 100, the second tube body 200 and the third tube body 300 are disposed; alternatively, they may be arranged at an inclined angle.

In some embodiments, the two guide wire outlets 121 are spaced apart along the axial direction of the first tube body 100, and positioning markers are arranged opposite to the area between the two guide wire outlets 121. The two guide wire outlets 121 are spaced apart in the axial direction of the first tube body 100, the axial spacing between the two guide wire outlets 121 along the first tube body 100 may be greater than or equal to 0.5 mm, and increasing the axial spacing between the two guide wire outlets along the first tube body 100 facilitates puncture by the puncture guide wire. The guide wire outlets 121 may be circular, elliptical or other shapes; the embodiment shown in FIG. 2 adopts an elliptical shape.

The two guide wire outlets 121 may be circumferentially distributed at an angle of 180° on the first tube body 100. When the guide wire outlets 121 are elliptical, the minor axis length of each guide wire outlet 121 is greater than or equal to 0.3 mm; when the guide wire outlets 121 are circular, the bore diameter of each guide wire outlet 121 is greater than or equal to 0.3 mm. For example, the inner diameter of the guide wire outlet 121 may be set to Ø0.014" (0.36 mm). The axial spacing between the two guide wire outlets along the first tube body 100 may be determined according to practical requirements and is not limited herein.

After the three-lumen microcatheter provided in the embodiments of the present disclosure is delivered between the vascular intima A and media B and rotated, one of the two guide wire outlets 121 faces the vascular intima A while another faces the vascular media B. The puncture guide wire can penetrate the intima A but cannot penetrate the media B, so the guide wire outlet 121 oriented toward the intima can be identified based on whether penetration is successfully achieved.

In some embodiments, the second tube body 200 and the third tube body 300 are disposed on two sides of the first tube body 100, and the positioning markers are arranged on the second tube body 200 and the third tube body 300 to position the guide wire outlet 121 of the first tube body 100, enabling the puncture guide wire to pass through the guide wire outlet 121 when the guide wire outlet 121 faces the vascular intima A.

In some embodiments, the positioning markers include a first radiopaque marker 410 and a second radiopaque marker 420; and the first radiopaque marker 410 is disposed on the second tube body 200, and the second radiopaque marker 420 is disposed on the third tube body 300; each of the first radiopaque marker 410 and the second radiopaque marker 420 may be an annular member. The first radiopaque marker 410 and the second radiopaque marker 420 correspond to the two guide wire outlets 121, and cooperate with each other to position the position and orientation of the two guide wire outlets 121.

The materials of the first radiopaque marker 410 and/or the second radiopaque marker 420 may be radiopaque substances, such as resin doped with radiopaque metals including tungsten, bismuth and barium, or radiopaque metallic materials including platinum-iridium alloy and gold.

In some embodiments, the first radiopaque marker 410 and the second radiopaque marker 420 correspond to the area between the two guide wire outlets 121 and are symmetrically arranged relative to the first tube body 100. In other embodiments, the first radiopaque marker 410 and the second radiopaque marker 420 correspond to the area between the two guide wire outlets 121 and are arranged in a staggered manner.

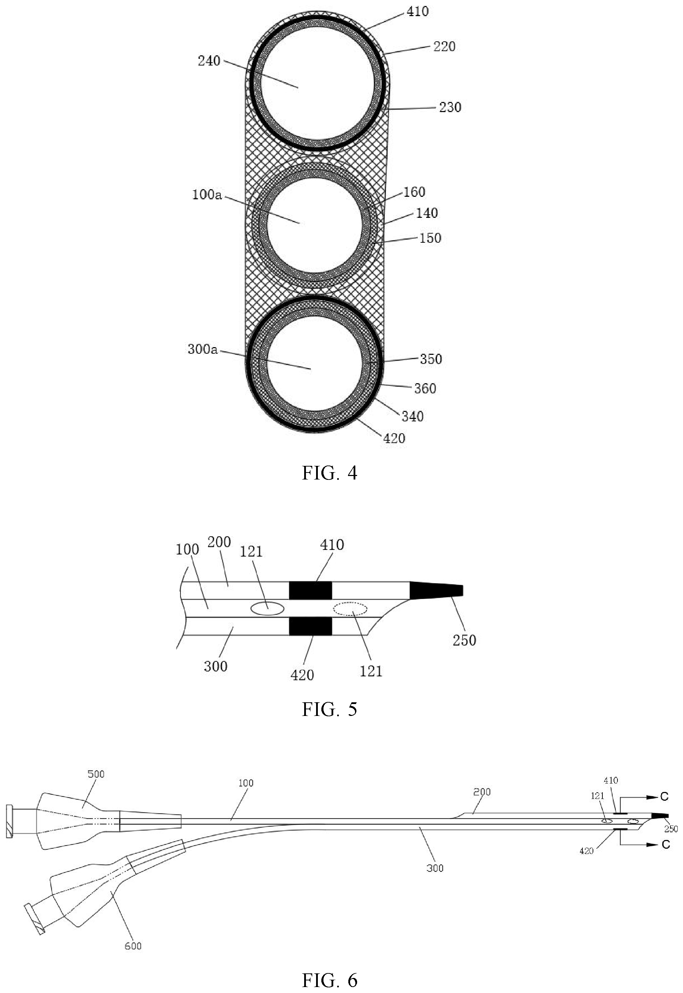

During positioning, the three-track or single-track state of the three-lumen microcatheter toward the intima is determined by observing the first radiopaque marker 410 and the second radiopaque marker 420. When irradiated from one direction by a fluoroscopic imaging device, if the first radiopaque marker 410 and the second radiopaque marker 420 overlap, the microcatheter is in a single-track state as shown in FIG. 5; if the first radiopaque marker 410 and the second radiopaque marker 420 are separated, the microcatheter is in a three-track state as shown in FIG. 4. When the three-track sign faces the intima A, puncture may be performed. Alternatively, if the three-lumen microcatheter is delivered with an unfavorable position and orientation, appropriate torsion may still be applied to ensure successful puncture.

As illustrated in FIG. 2 to FIG. 5, the positioning markers are radiopaque marker rings with an annular structure.

In some embodiments, as shown in FIG. 3 and FIG. 4, the guide wire lumen 100a of the first tube body 100 may be an OTW (Over-the-Wire) guide wire lumen. The first tube body 100 adopts a three-layer structure comprising an outer tube layer 140, an intermediate reinforcing layer 150 and a smooth inner layer 160, which allows passage of the puncture guide wire as well as aspiration of hematoma, fluid and the like. The inner cavity 240 of the second tube body 200 may be a rapid exchange lumen, and the second tube body 200 may adopt a two-layer structure design including an outer layer 220 and an inner layer 230 to facilitate delivery and withdrawal of instruments and save procedural time. The lumen 300a of the third tube body 300 may serve as an aspiration lumen for intraoperative aspiration of hematoma, fluid and the like, and the third tube body 300 comprises an outer layer 340, an intermediate reinforcing layer 360 and an inner layer 350. The inner layer may be made of PTFE (Poly tetra fluoroethylene) or other smooth resin materials, and the intermediate reinforcing layer may adopt a metallic braid and/or metallic spring structure.

As shown in FIG. 2 and FIG. 5, in some embodiments, a radiopaque distal tip 250 is disposed at the distal end of the second tube body 200, the distal tip 250 extends further distally relative to the first tube body 100 and the third tube body 300, and the distal tip 250 enables radiopaque positioning during surgery.

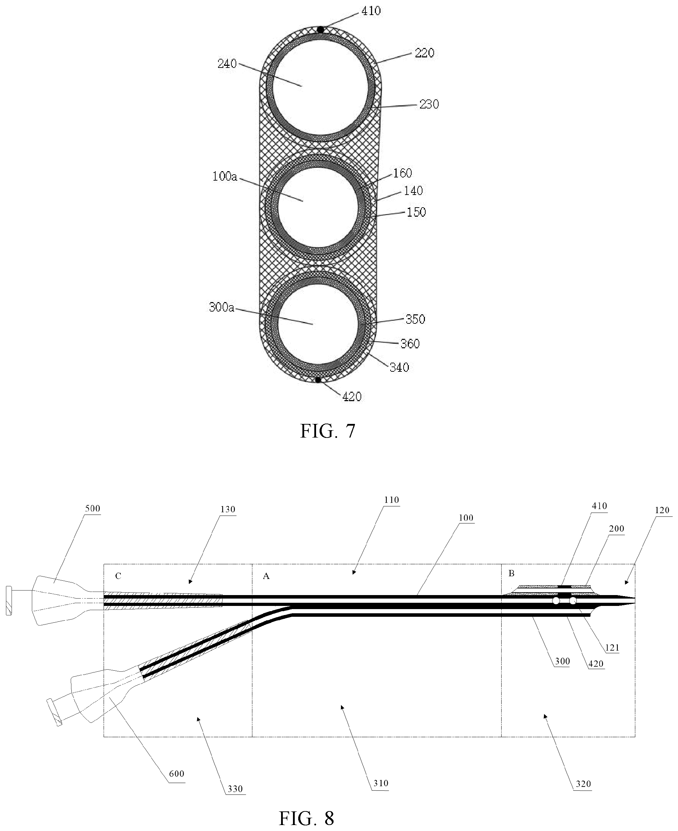

As shown in FIG. 6 and FIG. 7, another three-lumen microcatheter according to an embodiment of the present disclosure is provided, the positioning markers are radiopaque marker wires, radiopaque marker wires are respectively arranged on the second tube body 200 and the third tube body 300, the two radiopaque marker wires are respectively a first radiopaque marker 410 and a second radiopaque marker 420, the first radiopaque marker 410 is disposed on the second tube body 200, and the second radiopaque marker 420 is disposed on the third tube body 300.

The first radiopaque marker 410 is arranged on a side of the second tube body 200 facing away from the first tube body 100, and the first radiopaque marker 410 is parallel to the central axis of the second tube body 200. The second radiopaque marker 420 is arranged on a side of the third tube body 300 facing away from the first tube body 100, and the second radiopaque marker 420 is parallel to the central axis of the third tube body 300.

As shown in FIG. 8 to FIG. 10, another three-lumen microcatheter according to an embodiment of the present disclosure is provided, the first tube body 100 includes a connected proximal tube segment 110 (located in Region A of FIG. 8) and a distal tube segment 120 (located in Region B of FIG. 8), and the guide wire lumen 100a penetrates the proximal tube segment 110 and the distal tube segment 120. The guide wire outlets 121 are formed on side walls of the distal tube segment 120, a puncture guide wire can travel inside the guide wire lumen 100a, and the puncture guide wire can extend out of the first tube body 100 through the guide wire outlets 121.

It should be noted that the proximal end mentioned in the embodiments of the present disclosure refers to the left end shown in FIG. 8.

With reference to FIG. 8, the third tube body 300 may include a connected proximal tube segment 310 (a portion of the third tube body 300 arranged in Region A) and a distal tube segment 320 (a portion of the third tube body 300 arranged in Region B), a lumen 300a is formed inside the third tube body 300, and the lumen 300a penetrates the proximal tube segment 310 and the distal tube segment 320. The proximal tube segment 310 and the proximal tube segment 110 are arranged side by side in a flattened configuration, the distal tube segment 320, the distal tube segment 120 and the second tube body 200 are arranged side by side in a flattened configuration, that is, the first tube body 100 and the third tube body 300 are connected in parallel, and the second tube body 200, the distal tube segment 120 of the first tube body and the distal tube segment 320 of the third tube body are also connected in parallel. The first tube body 100, the second tube body 200 and the third tube body 300 may be integrally formed, and they may certainly be connected by welding, adhesive bonding or other methods.

In some embodiments, both the guide wire lumen 100a inside the first tube body 100 and the lumen inside the third tube body 300 may be OTW (Over-the-Wire) guide wire lumens, and lumens of other structures are certainly also acceptable as long as the guide wire lumen 100a inside the first tube body 100 allows passage of puncture guide wires and guiding guide wires as well as aspiration of hematoma and the like, and the lumen inside the third tube body 300 enables aspiration of hematoma and the like. The lumen inside the second tube body 200 may adopt any structure and is not limited herein.

It can be understood that aspiration ports are disposed at distal ends of the first tube body 100 and the third tube body 300, and lumens inside the first tube body 100 and the third tube body 300 aspirate hematoma, fluid and the like through the aspiration ports.

During surgery, the guide wire lumen 100a of the first tube body 100 may accommodate guiding guide wires and puncture guide wires, and lumens inside the first tube body 100 and/or the third tube body 300 may be used to aspirate hematoma, fluid and the like.

With reference to FIG. 8, FIG. 14 and FIG. 15, during operation, a guiding guide wire is placed inside the guide wire lumen 100a of the first tube body 100, the guiding guide wire first advances between the vascular intima A and media B, then the three-lumen microcatheter of the embodiment of the present disclosure advances along the guiding guide wire into the space between the intima A and media B, after reaching the space between the vascular intima A and media B, the three-lumen microcatheter deflects under force, the guide wire outlet in the guide wire lumen 100a of the deflected first tube body 100 faces the vascular intima A, then the guiding guide wire is withdrawn from the guide wire lumen 100a, next the puncture guide wire is delivered into the guide wire lumen 100a of the first tube body 100, the puncture guide wire penetrates the intima through the guide wire outlet 121, upon completion of puncture, the three-lumen microcatheter is withdrawn from the patient along the puncture guide wire, and the puncture guide wire remains inside the patient's body to assist subsequent procedures. During surgery, clinicians may simultaneously aspirate hematoma, fluid and the like through lumens of the third tube body 300 and the first tube body 100 to reduce procedural time. Of course, hematoma, fluid and the like may also be aspirated only through the lumen inside the third tube body 300 or the first tube body 100 according to surgical requirements.

During surgery, the guide wire lumen 100a of the first tube body 100 may accommodate the puncture guide wires, The lumen inside the third tube body 300 is configured to pass a guiding guide wire, and lumens inside the first tube body 100 and/or the third tube body 300 may be used to aspirate hematoma, fluid and the like.

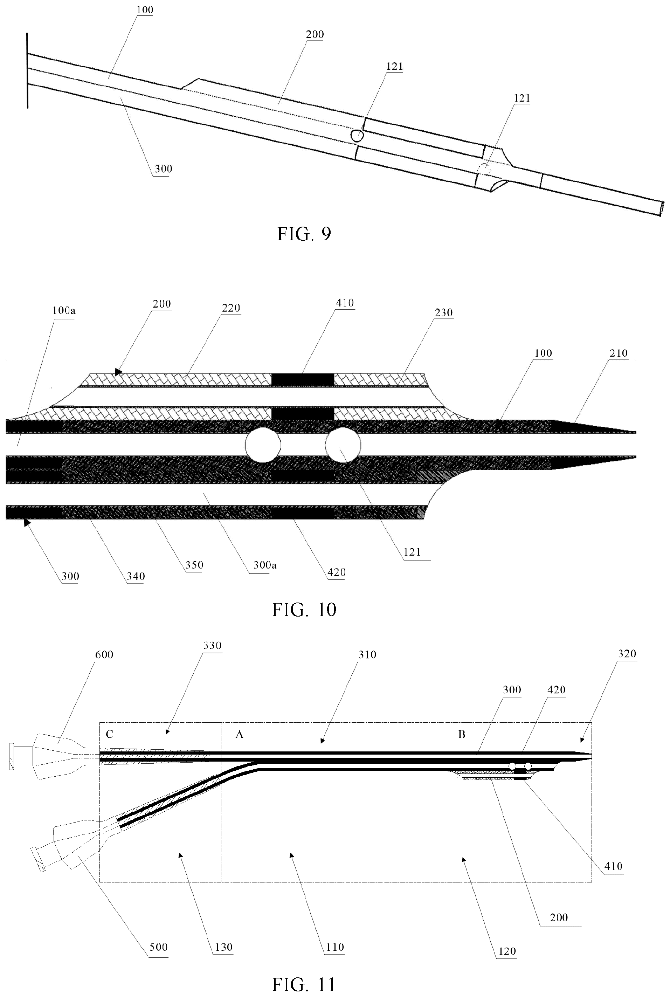

With reference to FIG. 11 to FIG. 13, another three-lumen microcatheter is provided according to an embodiment of the present disclosure, during operation, a guiding guide wire is arranged inside the lumen 300a of the third tube body 300, the guiding guide wire first enters the space between the vascular intima A and media B, then the three-lumen microcatheter according to the embodiment of the present disclosure enters the space between the intima A and media B along the guiding guide wire, the three-lumen microcatheter deflects under force after arriving between the vascular intima A and media B, the guide wire outlet in the guide wire lumen of the deflected first tube body 100 faces the vascular intima, after the three-lumen microcatheter arrives between the vascular intima A and media B, the guiding guide wire is withdrawn from the lumen inside the third tube body 300, then the puncture guide wire enters the guide wire lumen of the first tube body 100, the puncture guide wire penetrates the intima through the guide wire outlet 121 and enters the distal lesion site within the true vascular lumen, after the puncture by the puncture guide wire is completed, the three-lumen microcatheter is withdrawn from the human body along the puncture guide wire, and the puncture guide wire remains in the human body to assist subsequent surgical procedures. During surgery, clinicians may simultaneously aspirate hematoma, fluid and the like through lumens of the third tube body 300 and the first tube body 100 to reduce procedural time. Of course, hematoma, fluid and the like may also be aspirated only through the lumen inside the third tube body 300 or the first tube body 100 according to surgical requirements.

In some embodiments, the guide wire lumen 100a inside the first tube body 100 may be an OTW (Over-the-Wire) lumen for passage of a puncture guide wire and aspiration of hematoma, fluid and the like. The lumen inside the third tube body 300 may also be an OTW (Over-the-Wire) lumen for aspiration of hematoma, fluid and the like. The lumen inside the second tube body 200 may be a rapid exchange lumen (abbreviated as RX) for passage of a guiding guide wire. Certainly, the lumens inside the first tube body 100, the second tube body 200 and the third tube body 300 may adopt other structures as long as the above functions can be realized.

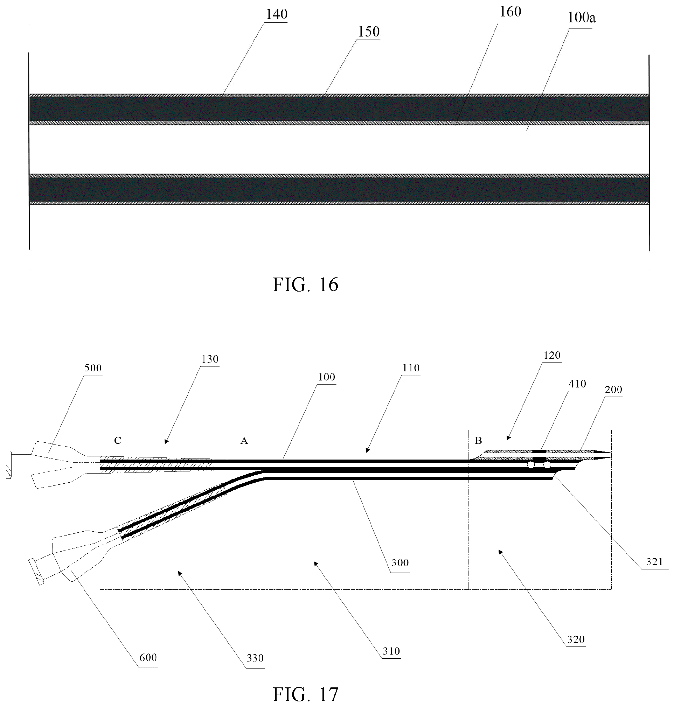

It can be understood that aspiration ports 321 are disposed at the distal ends of the first tube body 100 and the third tube body 300, with reference to FIG. 17, another three-lumen microcatheter is provided according to an embodiment of the present disclosure, and the lumens inside the first tube body 100 and the third tube body 300 aspirate hematoma, fluid and the like through the aspiration ports.

During surgery, the guide wire lumen 100a of the first tube body 100 is used to pass a puncture guide wire, and the lumen inside the second tube body 200 is used to pass a guiding guide wire. The lumens inside the first tube body 100 and/or the third tube body 300 may be used to aspirate hematoma, fluid and the like.

With reference to FIG. 17, during operation, a guiding guide wire is arranged inside the lumen of the second tube body 200, the guiding guide wire first enters the space between the vascular intima A and media B, then the three-lumen microcatheter according to the embodiment of the present disclosure enters the space between the intima A and media B along the guiding guide wire, the three-lumen microcatheter deflects under force after arriving between the vascular intima A and media B, the guide wire outlet in the guide wire lumen 100a of the deflected first tube body 100 faces the vascular intima, after the three-lumen microcatheter arrives between the vascular intima A and media B, the guiding guide wire is withdrawn from the lumen inside the second tube body 200, then the puncture guide wire enters the guide wire lumen 100a of the first tube body 100, the puncture guide wire penetrates the intima through the guide wire outlet 121 and enters the distal lesion site within the true vascular lumen, after the puncture by the puncture guide wire is completed, the three-lumen microcatheter is withdrawn from the human body along the puncture guide wire, and the puncture guide wire remains in the human body to assist subsequent surgical procedures. During surgery, clinicians may simultaneously aspirate hematoma, fluid and the like through lumens of the third tube body 300 and the first tube body 100 to reduce procedural time. Of course, hematoma, fluid and the like may also be aspirated only through the lumen inside the third tube body 300 or the first tube body 100 according to surgical requirements.

The reason why the three-lumen microcatheter deflects inside the intramembranous vascular space is described as follows with reference to FIG. 14, the first tube body 100, the second tube body 200 and the third tube body 300 are arranged side by side in a flattened configuration rather than assembled to form a circular tube, when the three-lumen microcatheter enters the intramembranous space and assumes the state shown in FIG. 14, this state is unstable for two reasons, on one hand, the two tube bodies located at the uppermost and lowermost positions relative to the state shown in FIG. 14 are in line contact with the intima A and media B, and each of the upper and lower tube bodies only forms a single contact line with the intima A and the media B respectively, on the other hand, the intima A and the media B exert a certain pressure (force in direction C shown in FIG. 14 and FIG. 15) on the three-lumen microcatheter, based on the above two reasons, the three-lumen microcatheter deflects to reach the stable state shown in FIG. 15.

In addition, the parallel design of the first tube body 100 and the third tube body 300 in the present embodiment improves the trackability of the three-lumen microcatheter, and powerful puncture against the vascular intima can be performed using one guide wire while realizing rapid guide wire exchange. Furthermore, clinicians may directly aspirate hematoma through the lumen 300a inside the third tube body 300 to shorten procedural time.

In some embodiments, with reference to FIG. 8, the second tube body 200 and the third tube body 300 are respectively disposed on two sides of the first tube body 100, and the distal end of the first tube body 100 is positioned further distally than the distal ends of the second tube body 200 and the third tube body 300, which facilitates passage of the three-lumen microcatheter inside blood vessels and improves the crossability of the three-lumen microcatheter.

The distal ends of the second tube body 200 and the third tube body 300 are formed as inclined surfaces, which effectively reduces the cross-sectional area of the distal portion of the three-lumen microcatheter and further improves the crossability of the three-lumen microcatheter inside blood vessels.

In some embodiments, a positioning member 210 may be fixed at the distal end of the first tube body 100. The positioning member 210 may be a radiopaque marker for tracking and locating the distal portion of the second tube body 200.

In some embodiments, as shown in FIG. 11 to FIG. 13, the distal ends of the second tube body 200, the first tube body 100 and the third tube body 300 are arranged in a stepped configuration, that is, the distal ends of the second tube body 200, the first tube body 100 and the third tube body 300 are disposed at inclined arrangement, which effectively reduces the cross-sectional area of the distal portion of the three-lumen microcatheter, facilitates passage of the three-lumen microcatheter inside blood vessels, and improves the crossability of the three-lumen microcatheter.

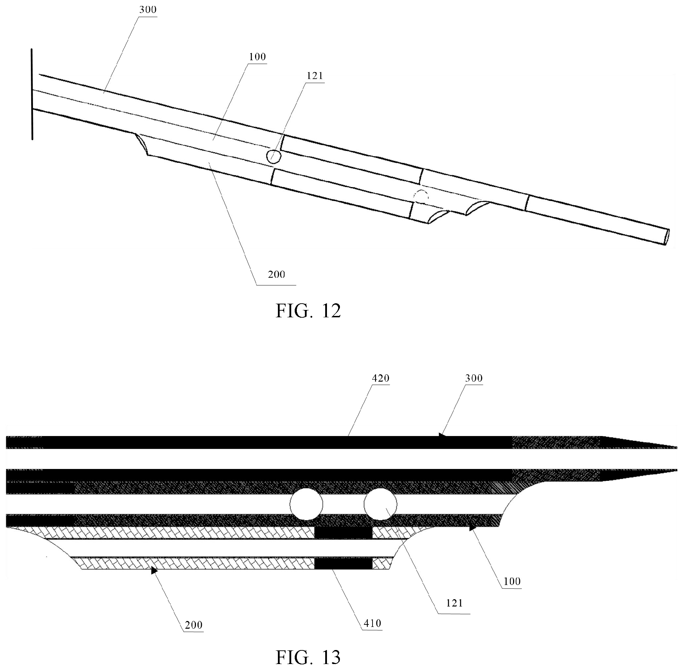

The second tube body 200 and the third tube body 300 may be disposed on two sides of the first tube body 100, the distal end of the second tube body 200 is positioned at the farthest distal position of the stepped arrangement (the rightmost position shown in FIG. 8 to FIG. 9), or the distal end of the third tube body 300 is positioned at the farthest distal position of the stepped arrangement (the rightmost position shown in FIG. 12).

In some embodiments, when the distal end of the second tube body 200 is at the farthest distal position of the stepped arrangement, the outer diameter of the distal end of the second tube body 200 gradually decreases from the proximal end to the distal end. The distal ends of the first tube body 100 and the third tube body 300 may be formed as inclined surfaces, that is, the tip portions of the guide wire lumen inside the first tube body 100 and the lumen inside the third tube body 300 are inclined to further improve the crossability of the three-lumen microcatheter according to the embodiments of the present disclosure.

A positioning member 210 may be fixed at the distal tip portion of the second tube body 200. The positioning member 210 may be a radiopaque marker for tracking and locating the distal portion of the second tube body 200.

In some embodiments, as shown in FIG. 11 to FIG. 13, when the distal end of the third tube body 300 is at the farthest distal position of the stepped arrangement, the outer diameter of the distal end of the third tube body 300 gradually decreases from the proximal end to the distal end, the distal ends of the first tube body 100 and the second tube body 200 are formed as inclined surfaces, that is, the tip portions of the guide wire lumen inside the first tube body 100 and the lumen inside the third tube body 300 are inclined to further improve the crossability of the three-lumen microcatheter according to the embodiments of the present disclosure.

A positioning member 210 may be fixed to the distal portion of the third tube body 300. The positioning member may be a radiopaque marker for tracking and locating the distal portion of the third tube body 300. Preferably, the radiopaque marker may be a flexible radiopaque member.

In some embodiments, the first tube body 100 further includes a stress relief tube segment 130 (located in Region C of FIG. 8), one end (the right end shown in FIG. 8) of the stress relief tube segment 130 is connected to the proximal tube segment 110, another end (the left end shown in FIG. 8) is configured to connect with a catheter hub 500, and the stress relief tube segment 130, the proximal tube segment 110 and the distal tube segment 120 communicate with each other to form a guide wire lumen.

In some embodiments, the stress relief tube segment 130 may be made of polyester elastomer or the like, and the catheter hub 500 may be made of resin material such as polycarbonate or the like.

In the present embodiment, the tail portion of the OTW lumen inside the first tube body 100 is docked with the catheter hub 500 to facilitate guide wire exchange, reagent injection, hematoma aspiration and the like, as well as connection with other instruments. In addition, a transition is formed by the stress relief tube segment 130 between the proximal end of the OTW lumen and the catheter hub 500 to avoid bending caused by stress concentration.

The hardness of the stress relief tube segment 130, the proximal tube segment 110 and the distal tube segment 120 decreases sequentially, which can prevent damage to blood vessels and other tissues while guaranteeing overall pushability.

In some embodiments, the third tube body 300 further includes a stress relief tube segment 330 (a portion of the third tube body 300 arranged in Region C of FIG. 11), one end (the right end shown in FIG. 11) of the stress relief tube segment 330 is connected to the proximal tube segment 310, another end (the left end shown in FIG. 11) of the stress relief tube segment 330 is configured to connect with a catheter hub 600, and the inner spaces of the stress relief tube segment 330, the proximal tube segment 310 and the distal tube segment 320 communicate with each other to form an aspiration lumen.

The stress relief tube segment 330 may be made of polyester elastomer or the like, and the catheter hub 600 may be made of resin material such as polycarbonate or the like.

In the present embodiment, the tail portion of the OTW lumen inside the third tube body 300 is docked with the catheter hub 600 to facilitate guide wire exchange, reagent injection, hematoma aspiration and the like, as well as connection with other instruments. In addition, a transition is formed by the stress relief tube segment 330 between the proximal end of the OTW lumen and the catheter hub 600 to avoid bending caused by stress concentration.

Wherein, the hardness of the stress relief tube segment 330, the proximal tube segment 310 and the distal tube segment 320 of the third tube body 300 decreases sequentially, which can prevent damage to blood vessels and other tissues while ensuring overall pushability.

The first tube body 100 can be used for passing a puncture guide wire and aspirating edema, blood clots, fluid and the like. During surgery, the first tube body 100 and the third tube body 300 can simultaneously aspirate edema, blood clots and the like to further shorten the operation time.

With reference to FIG. 16, the OTW lumen of the first tube body 100 may adopt a three-layer structure including an outer tube layer 140, an intermediate reinforcing layer 150 and a smooth inner layer 160 for passing a puncture guide wire and aspirating hematoma, fluid and the like. The rapid exchange lumen of the second tube body 200 may adopt a two-layer structure including an outer layer 220 and an inner layer 230 to facilitate delivery and withdrawal of instruments and save operation time. The third tube body 300 includes an outer layer 340 and an inner layer 350. The inner layer may be made of PTFE (Poly tetra fluoroethylene) or other smooth resin materials, and the intermediate reinforcing layer may adopt a metal braided structure and/or a metal spring structure.

Even if the three-lumen microcatheter is delivered to an unfavorable position and orientation, that is, the guide wire outlet 121 fails to face the vascular intima with a certain deviation, the configuration of the three-lumen microcatheter and the reinforcing layer (spring and/or braided layer) provided on the first tube body 100 allow proper torsion to orient the guide wire outlet 121 toward the intima, which raises the success rate for the puncture guide wire to re-enter the true vascular lumen and thus maximizes the success rate of a single operation.

Various embodiments of the present disclosure have been described above, and the foregoing description is illustrative rather than exhaustive and is not limited to the disclosed embodiments. Many modifications and variations will be apparent to those of ordinary skill in the art without departing from the scope and spirit of the described embodiments. The selection of terms used herein is intended to best explain the principles of the embodiments, practical applications, or improvements to technologies on the market, or to enable other ordinary skilled persons in the art to understand the embodiments disclosed herein.

## Claims

1. A three-lumen microcatheter, comprising:
a first tube body, wherein a guide wire lumen is formed inside the first tube body;
a second tube body; and
a third tube body;
wherein the first tube body has at least one guide wire outlet formed in a side wall of a distal end of the first tube body, and the at least one guide wire outlet communicating with the guide wire lumen;
wherein the second tube body, a distal portion of the first tube body and a distal portion of the third tube body are arranged side by side in a flattened configuration, so that the at least one guide wire outlet faces a vascular intima and/or a media when the three-lumen microcatheter has entered a subintimal space; and
a positioning marker, wherein the second tube body and/or the third tube body is provided with the positioning marker.

2. The three-lumen microcatheter according to claim 1, wherein
axes of the first tube body, the second tube body and the third tube body are parallel to each other, and the first tube body, the second tube body and the third tube body lie in a same plane.

3. The three-lumen microcatheter according to claim 2, wherein
there are two guide wire outlets, the two guide wire outlets are formed in opposite side walls of the first tube body, and axes of the two guide wire outlets are perpendicular to the plane.

4. The three-lumen microcatheter according to claim 3, wherein
the two guide wire outlets are spaced apart along an axial direction of the first tube body; and
the positioning marker is arranged opposite to an area between the two guide wire outlets.

5. The three-lumen microcatheter according to claim 1, wherein
the positioning marker comprises:
a first radiopaque marker arranged on the second tube body; and
a second radiopaque marker arranged on the third tube body, wherein the first radiopaque marker and the second radiopaque marker correspond to the at least one guide wire outlet, respectively, and the first radiopaque marker cooperates with the second radiopaque marker to position the at least one guide wire outlet.

6. The three-lumen microcatheter according to claim 5, wherein
the first radiopaque marker and the second radiopaque marker are symmetrically arranged relative to the first tube body.

7. The three-lumen microcatheter according to claim 1, wherein
the positioning marker is a radiopaque marker ring or a radiopaque marker wire.

8. The three-lumen microcatheter according to claim 1, wherein
the first tube body comprises a proximal tube segment and a distal tube segment connected to each other, and the at least one guide wire outlet is arranged in a side wall of the distal tube segment; and
the third tube body comprises a proximal tube body and a distal tube body connected to each other, the proximal tube body is arranged side by side with the proximal tube segment, and the distal tube body, the distal tube segment and the second tube body are arranged side by side in a flattened configuration.

9. The three-lumen microcatheter according to claim 8, wherein
distal ends of the first tube body, the second tube body and the third tube body are arranged in a stepped arrangement.

10. The three-lumen microcatheter according to claim 9, wherein
the second tube body and the third tube body are disposed on two sides of the first tube body, and a distal end of the second tube body is located at a distal-most position of the stepped arrangement, or a distal end of the third tube body is located at the distal-most position of the stepped arrangement.

11. The three-lumen microcatheter according to claim 10, wherein
when the distal end of the second tube body is located at the distal-most position of the stepped arrangement, a cross-sectional area of the distal end of the second tube body gradually decreases in a direction from a proximal end to a distal end, and the distal ends of the first tube body and the third tube body each form an inclined surface; or
when the distal end of the third tube body is located at the distal-most position of the stepped arrangement, a cross-sectional area of the distal end of the third tube body gradually decreases in a direction from a proximal end to a distal end, and the distal ends of the first tube body and the second tube body each form an inclined surface.

12. The three-lumen microcatheter according to claim 8, wherein
the second tube body and the third tube body are respectively disposed on two sides of the first tube body, and a distal end of the first tube body protrudes beyond distal ends of the second tube body and the third tube body.

13. The three-lumen microcatheter according to claim 8, wherein
the first tube body further comprises a stress relief tube segment, one end of the stress relief tube segment is connected to the proximal tube segment, and another end is configured to connect to a catheter hub, and inner spaces of the stress relief tube segment, the proximal tube segment and the distal tube segment communicate with each other to form the guide wire lumen; and/or
the third tube body further comprises a stress relief tube body, one end of the stress relief tube body is connected to the proximal tube body, and another end is configured to connect to a catheter hub, and inner spaces of the stress relief tube body, the proximal tube body and the distal tube body communicate with each other to form a lumen.
